# EUROPÄISCHE PATENTSCHRIFT

(11) **EP 2 468 353 B1**
(45) Veröffentlichungstag und Bekanntmachung des Hinweises auf die Patenterteilung: **17.05.2017**
(21) Anmeldenummer: 11193144.0
(22) Anmeldetag: 13.12.2011
(51) Int. Cl.: A61N 1/05, A61N 1/36, A61N 1/39, A61N 1/08, A61N 1/37

(54) **Implantierbares Gerät**
Implantable device
Appareil implantable

(30) Priorität: 21.12.2010 US 201061425253 P
(43) Veröffentlichungstag der Anmeldung: 27.06.2012
(73) Patentinhaber: BIOTRONIK SE & Co. KG, 12359 Berlin (DE)
(72) Erfinder: Büssing, Heinrich, 10317 Berlin (DE); Weiss, Ingo, 12435 Berlin (DE); Sghaier, Sami, 12047 Berlin (DE)
(74) Vertreter: Galander, Marcus

(56) Entgegenhaltungen:
- US-A1- 2004 124 838
- US-A1- 2004 263 172

## Beschreibung

Die Erfindung betrifft ein permanent oder temporär implantierbares Gerät mit einem langgestreckten elektrischen Leiter.

Solche Geräte, beispielsweise Elektrodenleitungen für die Elektrostimulation, haben den Nachteil, dass sich ihr elektrischer Leiter in einem Kernspintomografen erwärmen kann, weil die im Kernspintomografen herrschenden wechselnden Magnetfelder in dem elektrischen Leiter nicht unbeachtliche elektrische Ströme induzieren. Auch können solche induzierten Ströme über Elektrodenpole der Elektrodenleitung an umliegendes Gewebe abgegeben werden und so beispielsweise zu unerwünschten Gewebeerwärmungen führen. Deshalb können Herzschrittmacherpatienten heutzutage in der Regel nicht oder nur eingeschränkt in einem Kernspintomografen untersucht werden.

An implantierbaren Herzschrittmachern oder Defibrillatoren (im Folgenden auch gemeinsam als Herzstimulatoren oder IPG (implantable pulse generator) bezeichnet) sind nämlich typischerweise wenigstens eine Stimulationselektrodenleitung angeschlossen, die an ihrem proximalen, zum Anschluss an den Herzschrittmacher oder Defibrillator vorgesehenen Ende einen standardisierten elektrischen Anschluss aufweist und an ihrem distalen, zur Platzierung im Herzen vorgesehenen Ende einen oder mehrere Elektrodenpole aufweist. Ein solcher Elektrodenpol dient zur Abgabe elektrischer Impulse an das Gewebe (Myokard) des Herzens oder zum Abfühlen elektrischer Felder, um im Rahmen des sogenannten Sensings eine Aktivität eines Herzens abfühlen zu können. Zu diesen Zwecken bilden Elektrodenpole typischerweise elektrisch leitende Oberflächenabschnitte einer Elektrodenleitung. Elektrodenpole sind typischerweise als Ringelektrode in Form eines Rings um die Elektrodenleitung oder in Form einer Spitzen- oder Tippelektrode am distalen Ende der Elektrodenleitung vorgesehen. Die Elektrodenpole sind über eine oder mehrere elektrische Leiter mit Kontakten des elektrischen Anschlusses der Elektrodenleitung an deren proximalem Ende elektrisch leitend verbunden. Somit verlaufen zwischen den Kontakten des elektrischen Anschlusses die Elektrodenleitungen an deren proximalen Ende und den Elektrodenpolen am distalen Ende der Elektrodenleitung ein oder mehrere elektrische Leiter, die einen oder mehrere der Elektrodenpole mit einem oder mehreren der Kontakte elektrisch verbinden. Diese elektrischen Leiter können einerseits zur Übertragung von Stimulationsimpulsen zu den Elektrodenpolen und andererseits zur Übertragung mittels der Elektrodenpole aufgenommener elektrischer Signale zum proximalen Ende der Elektrodenleitung genutzt werden und werden im Verlauf der weiteren Beschreibung auch jeweils als Funktionsleitung bezeichnet. Solche Funktionsleitungen sind für die Funktionen der jeweiligen Elektrodenleitung erforderliche elektrische Leiter und als solche der Gefahr ausgesetzt, dass in ihnen durch äußere Wechselmagnetfelder elektrische Ströme induziert werden, die beispielsweise zu einer unerwünschten Erwärmung der Funktionsleitungen oder der mit ihr verbundenen Elektrodenpole führen können oder die zur Abgabe entsprechender Ströme über die Elektrodenpole an umgebendes Gewebe und damit zu einer Erwärmung des umgebenden Gewebes führen können.

Die US 2004/263172 beschreibt eine Vorrichtung zur Verminderung der Auswirkungen von durch wechselnde Magnetfelder in einen Apparat induzierten Spannungen, bestehend aus einem Sensor zur Messung von in die leitenden Teile eines Apparates induzierten Spannungen und einer mit dem Sensor verbundenen aktiven oder passiven Kompensationsschaltung, wobei die Kompensation durch eine Spannung mit gegenüber der induzierten Spannung entgegengesetztem Vorzeichen erfolgt.

Der Erfindung liegt die Aufgabe zugrunde, ein Gerät zu schaffen, welches das zuvor beschriebene Problem löst.

Erfindungsgemäß wird diese Aufgabe durch ein Gerät gelöst, welches mit wenigstens einem langgestreckten elektrischen Funktionsleiter für die Übertragung von Therapiesignalen oder Diagnosesignalen oder beidem verbunden oder zu verbinden ist und eine steuerbare Spannungs- oder Stromquelle (als Kompensationssignalgenerator) oder eine einstellbare Abschlussimpedanz für den Funktionsleiter aufweist, die mit dem Funktionsleiter verbunden oder zu verbinden ist, sowie
eine Steuereinheit, die mit der Spannungs- oder Stromquelle oder der einstellbaren Abschlussimpedanz verbunden und ausgebildet ist, eine von der Spannungs- oder Stromquelle auf die Funktionsleitung aufzuprägende Spannung oder einen aufzuprägenden Strom zu steuern oder die Abschlussimpedanz einzustellen, sowie
einen Störfeldsensor, der mit der Steuereinheit verbunden und ausgebildet ist, ein ggf. vorhandenes elektromagnetisches oder magnetisches Wechselfeld zu erfassen und ein einem erfassten elektromagnetischen oder magnetischen Wechselfeld entsprechendes Ausgangssignal an die Steuereinheit zu liefern.

Hierbei ist die Steuereinheit ausgebildet, die Spannungs- oder Stromquelle in Abhängigkeit des Ausgangssignals des Störfeldsensors hinsichtlich einer ggf. auf die Funktionsleitung aufzuprägenden Spannung oder eines aufzuprägenden Stroms so zu steuern oder die einstellbare Impedanz so einzustellen, dass durch Überlagerung eines von der Strom- oder Spannungsquelle generierten oder infolge der einstellbaren Impedanz reflektierten Signals eine durch ein ggf. vorhandenes elektromagnetisches oder magnetisches Wechselfeld induzierte Spannung am distalen Ende der Elektrodenleitung zu kompensieren.

Die Erfindung schließt als ein erfinderisches Prinzip den Gedanken ein, am proximalen Ende ein entgegenwirkendes Signal aktiv oder passiv so zu induzieren, dass eine Erwärmung der Spitze teilweise oder völlig zu verhindert wird.

Hierzu wird an einem distalen Ende eines langgestreckten Implantats wie beispielsweise einer Elektrodenleitung ein Detektor platziert, der ein Signal über die Erwärmung der Spitze ans proximale Ende zurückleitet, um ggf. den Regelkreis zum Kompensieren der an der Spitze herrschenden Felder zu vervollständigen.

Im Falle eines Gerätes, das eine Elektrodenleitung aufweist ist es bevorzugt, wenn die einstellbare Impedanz oder die Spannungsquelle an oder in der Nähe eines proximalen Endes der Elektrodenleitung angeordnet ist.

Eine bevorzugte einstellbare Abschlussimpedanz enthält ein Bauteil, mit dem ein Blindwiderstand und ein Bauteil, mit dem ein Wirkwiderstand eingestellt werden kann. Das Bauteil, mit dem ein Blindwiderstand eingestellt werden kann ist vorzugsweise eine Kapazitätsdiode. Das Bauteil, mit dem ein Wirkwiderstand eingestellt werden kann ist vorzugsweise eine PIN-Diode.

Eine bevorzugte Spannungsquelle weist einen spannungsgesteuerten Oszillator auf.

Der Störfeldsensor ist vorzugsweise dazu ausgebildet, eine am proximalen Ende einer Elektrodenleitung empfangene Leistung zu erfassen.

Alternativ kann der Störfeldsensor am distalen Ende einer Elektrodenleitung angeordnet sein und ein nichtlineares Bauteil aufweisen, das bei Stromfluss oder anliegender Spannung Oberwellen generiert, die dann von einer Steuereinheit am proximalen Ende der Elektrodenleitung erfasst und zur Steuerung der Spannungsquelle oder der einstellbaren Impedanz ausgewertet werden können. Das nichtlineare Bauteil kann beispielsweise eine Diode sein.

Gemäß einer besonders bevorzugten Ausführungsvariante weist der Störfeldsensor ein nichtlineares Bauteil auf, das bei Stromfluss oder anliegender Spannung Oberwellen generiert und die Steuereinheit ist ausgebildet, die einstellbare Abschlussimpedanz oder die Spannungsquelle so einzustellen, dass diese Oberwellen verschwinden.

Die Erfindung soll nun anhand von Ausführungsbeispielen mit Bezug auf die Figuren näher erläutert werden. Die Figuren zeigen Folgendes:
- Fig. 1: zeigt als implantierbares medizinisches Gerät einen implantierbaren Herzstimulator 10 und eine daran angeschlossene implantierbare Elektrodenleitung 20.
- Fig. 2: zeigt eine Elektrodenleitung in die an verschiedenen Stellen elektrische Spannungen eingestreut werden.
- Fig. 3: zeigt ein implantierbares medizinisches Gerät mit daran angeschlossener Elektrodenleitung, die an ihrem proximalen Ende eine gesteuerte Spannungsquelle besitzt.
- Fig. 4A: zeigt einen Störfeldsensor mit einer Diode als nichtlinearem Bauteil, der auch an einem distalen Ende einer Elektrodenleitung angeordnet sein kann.
- Fig. 4B: zeigt ein Ausführungsbeispiel eines Störfeldsensors mit einem nichtlinearen Bauteil für eine Elektrodenleitung mit mehreren Elektrodenpolen.
- Fig. 5: zeigt ein Ausführungsbeispiel für einen einstellbaren Widerstand Zi als einstellbare Impedanz, der am proximalen Ende einer Elektrodenleitung oder im Gehäuse eines Herzstimulators eingebaut sein kann.

Der implantierbare Herzstimulator 10 kann ein Herzschrittmacher oder ein Kardioverter/Defibrillator (ICD) sein. Im dargestellten Ausführungsbeispiel ist der Herzstimulator 10 ein ventrikulärer Herzschrittmacher und Defibrillator. Andere bekannte Herzstimulatoren sind Zweikammerherzschrittmacher zur Stimulation des rechten Atriums und des rechten Ventrikels oder biventrikuläre Herzschrittmacher, die zusätzlich zum rechten Ventrikel auch den linken Ventrikel stimulieren können.

Derartige Stimulatoren besitzen typischerweise ein Gehäuse 12, das im Regelfall aus Metall besteht und somit elektrisch leitend ist und als großflächiger Elektrodenpol dienen kann. An der Außenseite des Gehäuses 12 ist typischerweise ein Anschlussgehäuse 14 befestigt, das auch als Header bezeichnet wird. Ein derartiger Header weist typischerweise Kontaktbuchsen zur Aufnahme von Steckkontakten auf. Die Kontaktbuchsen besitzen elektrische Kontakte 16, die über entsprechende Leiter mit einer in dem Gehäuse 12 des Herzstimulators 10 angeordneten Elektronik verbunden sind.

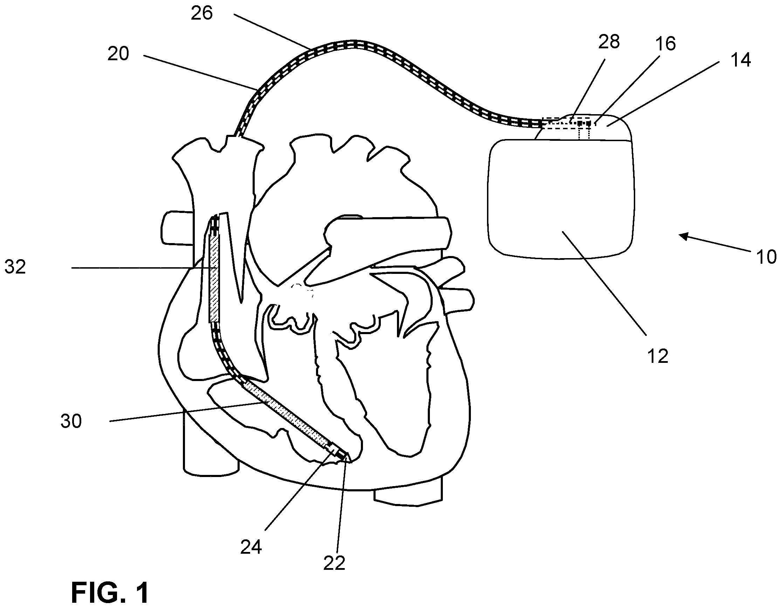

Die Elektrodenleitung 20 stellt im Sinne dieser Erfindung ebenfalls ein implantierbares medizinisches Gerät dar. Am distalen Ende der Elektrodenleitung 20 sind in an sich bekannter Manier Elektrodenpole in Form einer Spitzen- oder Tipelektrode 22 und einer in deren Nähe angeordneten Ringelektrode 24 angeordnet. Die Elektrodenpole 22 und 24 sind so ausgebildet, dass sie je nach Funktion eines Herzstimulators, an den die Elektrodenleitung 20 angeschlossen ist, zum Abfühlen elektrischer Potenziale des Herzgewebes (Myokards) dienen oder zur Abgabe elektrischer Signale, beispielsweise zur Abgabe von Stimulationsimpulsen an das sie umgebende Herzgewebe, ausgebildet sind. Fig. 1 zeigt, wie sich die Elektrodenpole, also die Tipelektrode 22 und die Ringelektrode 24, im Anwendungsfall die Elektrodenleitung 20, im Apex eines rechten Ventrikels eines Herzens befinden.

Sowohl die Tipelektrode 22 als auch die Ringelektrode 24 sind über jeweils wenigstens einen elektrischen Leiter 26 mit einem Steckkontakt 28 am proximalen Ende der Elektrodenleitung 20 elektrisch verbunden. Der Steckkontakt 28 besitzt elektrische Kontakte, die mit den elektrischen Kontakten 16 der Kontaktbuchse im Anschlussgehäuse 14 des implantierbaren Herzstimulators korrespondieren. Die elektrischen Leiter 26 in der Elektrodenleitung 20 können als annähernd gestreckte Seilzugleiter oder als helixförmig gewendelte Leiter ausgebildet sein. Solche Leiter, die funktionale Elektrodenpole mit elektrischen Kontakten des Steckkontaktes am proximalen Ende der Elektrodenleitung 20 elektrisch leitend verbinden werden im Rahmen dieses Textes als Funktionsleiter bezeichnet, da sie beispielsweise der Therapie dienende elektrische Signale von Steckkontakt zum jeweiligen Elektrodenpol übertragen oder abgefühlte elektrische Potentiale repräsentierende Signale vom jeweiligen Elektrodenpol zum Steckkontakt führen und somit der elementaren Funktion des medizinischen Gerätes dienen.

Die elektrischen Leiter 26, die die Elektrodenpole 22 bzw. 24 mit den elektrischen Kontakten des Steckers 28 der Elektrodenleitung 20 verbinden, sind über den größten Teil ihrer Länge von einer isolierenden Hülle umgeben, so dass ein elektrischer Kontakt zum Gewebe des Herzens gezielt über die Elektrodenpole zustande kommt.

Neben den Elektrodenpolen 22 und 24, die typischerweise der (in diesem Fall ventrikulären) Stimulation des Herzgewebes dienen, weist die Elektrodenleitung 20 auch noch zwei großflächigere Elektrodenpole 30 und 32 auf, die als Defibrillationselektroden dienen und von wenigstens einem blank liegendem helixartig gewendelten Draht gebildet sind.

Es sei darauf hingewiesen, dass die Erfindung im Rahmen dieses Ausführungsbeispiels anhand eines rechtsventrikulären Herzschrittmachers und Defibrillators erläutert wird. Als medizinisches Gerät im Sinne der Erfindung kann grundsätzlich aber beispielsweise auch eine Ablationselektrodenleitung dienen, die im Anwendungsfall ebenfalls bis ins Herz eines Patienten hineinragt und die von einem außerhalb des Patienten angeordneten Gerät gesteuert wird und hierzu mit diesem verbunden ist.

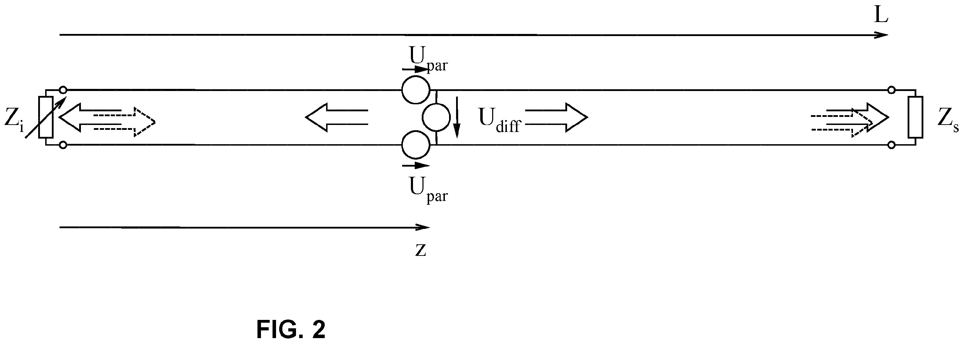

Figur 2 veranschaulicht, wie entlang einer Elektrodenleitung der Länge L durch äußere elektromagnetische Felder, z.B. bei MR-Untersuchungen, an verschiedenen Stellen z elektrische Spannungen (Uₚₐᵣ oder U_{diff}) in die Elektrodenleitung eingestreut werden. Dies führt zu einem Stromfluss (Wellenausbreitung) entlang der Elektrodenleitung in die Richtung zum Implantat und auch in die Richtung zum distalen Ende der Elektrodenleitung, im Folgenden auch als Spitze bezeichnet. An der Spitze kann der so induzierte Strom zu einer übermäßigen Erwärmung führen.

Da die Spannung in der Spitze der Elektrode durch die Überlagerung der Spannungen U_{diff} und Uₚₐᵣ an verschiedenen Stellen der Leitung entsteht und sich nach Phase, Dämpfung und Ausbreitungsgeschwindigkeit richtet (Übertragungsfunktion), ist es die der Erfindung zugrundeliegende Idee, durch Überlagerung eines zusätzlichen Signals im elektronischen Implantat die Spannung an der Elektrodenspitze - also am distalen Ende er Elektrodenleitung - auszugleichen.

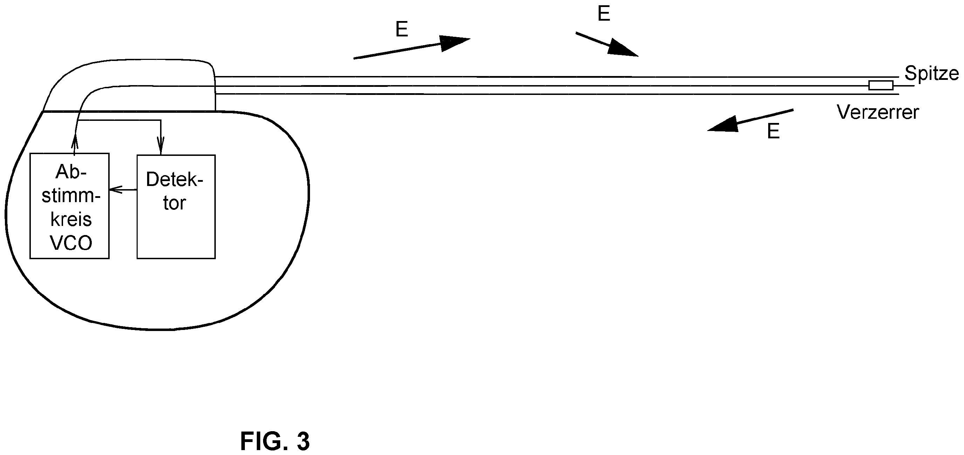

In Figur 2 ist der Widerstand des Implantats als einstellbarer Widerstand Zi dargestellt, der das eingestreute Signal (Pfeil) so reflektiert (gestrichelter Pfeil), dass es sich mit dem Signal an der Spitze zu null überlagert. Dies kann außer durch einen passiven einstellbaren Widerstand (Impedanz) auch durch eine einstellbare Spannungsquelle, insbesondere einen spannungsgesteuerten Oszillator (VCO, voltage controlled oscillator) realisiert sein, der sich am proximalen Ende befindet, wie dies in Figur 3 dargestellt ist.

Ein Störfeldsensor ist dazu vorgesehen, ein Ausgangssignal zu liefern, das eine Spannung, einen Strom oder eine Erwärmung der Spitze der Elektrodenleitung repräsentiert.

Gemäß einer Ausführungsvariante ist der Störfeldsensor dazu ausgebildet, dieses Signal aus einer am proximalen Ende der Elektrodenleitung empfangenen Leistung zu generieren. Der Störfeldsensor ist dann beispielsweise in das Gehäuse 12 des Herzstimulators 10 integriert.

Außer durch Detektion der empfangenen Leistung am proximalen Ende kann die Abstimmung des Widerstands Zi oder des Oszillators auch nach Spannung oder Strom in der Spitze durch ein geeignetes detektierendes oder sendendes Bauteil erfolgen, das dann den Störfeldsensor bildet. Z.B. kann ein nichtlineares Bauteil in die Spitze eingebaut werden, dass bei Stromfluss oder anliegender Spannung Signale (in diesem Beispiel Oberwellen) an das proximale Ende sendet. Der Widerstand oder Oszillator am proximalen Ende wird dann mittels eines von der Steuereinheit gebildeten Regelkreises so eingestellt, dass diese Oberwellen verschwinden. In diesem Fall bildet das jeweilige detektierende oder sendende Bauteil am distalen Ende der Elektrodenleitung den Störfeldsensor.

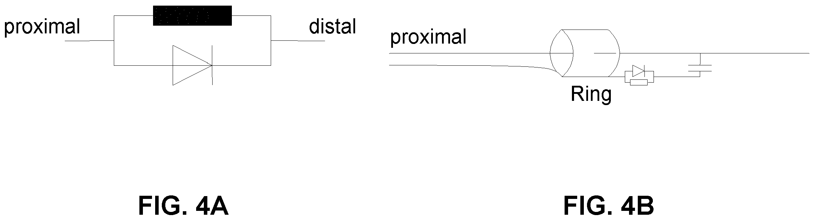

Figur 4A zeigt eine mögliche Ausführung. Hier wird eine Diode als nichtlineares Bauteil mit einer Spule in den Signalweg geschaltet und kann als Signal Oberwellen erzeugen, wenn sie von Strom durchflossen wird. Die Spule hat die Aufgabe, der Entstehung von Gleichspannung entgegenzuwirken, damit die Ladungsbilanz bei der Stimulation erhalten bleibt. Figur 4B zeigt ein weiteres Ausführungsbeispiel mit einem nichtlinearen Bauteil für eine Elektrodenleitung mit mehreren Elektrodenpolen. (z.B. Tip und Ring).

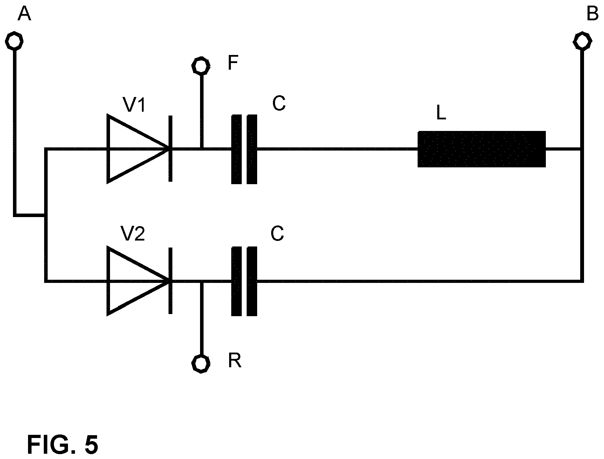

Figur 5 zeigt ein Ausführungsbeispiel für einen einstellbaren Widerstand Zi als einstellbare Impedanz zwischen den Kontakten A und B, der am proximalen Ende der Elektrodenleitung oder im Gehäuse des Herzstimulators eingebaut sein kann. V1 ist eine Kapazitätsdiode (sog. Varaktor), so dass durch Einstellung der Spannung am Anschluss F der Blindanteil von Zi eingestellt werden kann. V2 ist z.B. eine PIN-Diode, so dass durch Veränderung der Spannung am Anschluss R der Wirkwiderstand von Zi eingestellt werden kann.

Diese Entgegenwirkung funktioniert besonders gut, wenn die Dämpfung von Wellen auf der Elektrodenleitung gering ist, so dass am proximalen Ende viel Energie ankommt und eine starke Rückwirkung des proximalen Endes auf das distale Ende der Elektrodenleitung zu erwarten ist.

## Patentansprüche

1. Temporär oder permanent implantierbares medizinisches Gerät, aufweisend
einen implantierbaren Herzstimulator (10), sowie
eine Elektrodenleitung (20), die wenigstens einen langgestreckten elektrischen Funktionsleiter (26) umfasst, der für die Übertragung von Therapiesignalen oder Diagnosesignalen oder beidem mit dem Herzstimulator verbunden ist, sowie
eine steuerbare Spannungs- oder Stromquelle oder eine einstellbare Abschlussimpedanz für den Funktionsleiter (26), die mit dem Funktionsleiter (26) verbunden ist, sowie
eine Steuereinheit, die mit der Spannungs- oder Stromquelle oder der einstellbaren Abschlussimpedanz verbunden und ausgebildet ist, eine von der Spannungs- oder Stromquelle auf den Funktionsleiter (26) aufzuprägende Spannung oder einen aufzuprägenden Strom zu steuern oder die Abschlussimpedanz einzustellen, sowie einen Störfeldsensor, der mit der Steuereinheit verbunden und ausgebildet ist, ein ggf. vorhandenes elektromagnetisches oder magnetisches Wechselfeld zu erfassen und ein einem erfassten elektromagnetischen oder magnetischen Wechselfeld entsprechendes Ausgangssignal an die Steuereinheit zu liefern,
**dadurch gekennzeichnet, dass** am distalen Ende des verbundenen langgestreckten Funktionsleiters (26) ein Detektor platziert ist, der mit der Steuereinheit verbunden und ausgebildet ist, ein Signal über die Erwärmung des distalen Endes des Funktionsleiters (26) an die Steuereinheit zu liefern,
wobei die Steuereinheit ausgebildet ist, die Spannungs- oder Stromquelle in Abhängigkeit des Ausgangssignals des Störfeldsensors und des am distalen Ende platzierten Detektors hinsichtlich einer ggf. auf den Funktionsleiter (26) aufzuprägenden Spannung oder eines aufzuprägenden Stroms zu steuern oder die einstellbare Impedanz so einzustellen, dass durch Überlagerung eines von der Strom- oder Spannungsquelle generierten oder infolge der einstellbaren Impedanz reflektierten Signals eine durch ein ggf. vorhandenes elektromagnetisches oder magnetisches Wechselfeld induzierte Spannung am distalen Ende der Elektrodenleitung (20) zu kompensieren.

2. Gerät nach Anspruch 1, **dadurch gekennzeichnet, dass** die einstellbare Impedanz oder die Spannungsquelle an oder in der Nähe eines proximalen Endes der Elektrodenleitung (20) angeordnet ist.

3. Gerät nach Anspruch 1 oder 2, **dadurch gekennzeichnet, dass** die einstellbare Abschlussimpedanz eine Kapazitätsdiode und eine PIN-Diode enthält.

4. Gerät nach Anspruch 1 oder 2, **dadurch gekennzeichnet, dass** die Spannungsquelle einen spannungsgesteuerten Oszillator aufweist.

5. Gerät nach einem der Ansprüche 1 bis 4, **dadurch gekennzeichnet, dass** der Störfeldsensor dazu ausgebildet ist, eine am proximalen Ende einer Elektrodenleitung (20) empfangene Leistung zu erfassen.

6. Gerät nach einem der Ansprüche 1 bis 4, **dadurch gekennzeichnet, dass** der Störfeldsensor am distalen Ende einer Elektrodenleitung (20) angeordnet ist und ein nichtlineares Bauteil aufweist.

7. Gerät nach Anspruch 6, **dadurch gekennzeichnet, dass** das nichtlineare Bauteil eine Diode ist.

8. Gerät nach einem der Ansprüche 1 bis 7, **dadurch gekennzeichnet, dass** der Störfeldsensor ein nichtlineares Bauteil aufweist, das bei Stromfluss oder anliegender Spannung Oberwellen generiert und die Steuereinheit ausgebildet ist, die einstellbare Abschlussimpedanz oder die Spannungsquelle so einzustellen, dass diese Oberwellen verschwinden.

## Claims

1. A temporarily or permanently implantable medical device, comprising an implantable heart stimulator (10), and also
an electrode lead (20), which comprises at least one elongate electrical functional conductor (26), which is connected to the heart stimulator for the transfer of therapy signals or diagnosis signals or both, and also
a controllable voltage or current source or an adjustable terminating impedance for the functional conductor (26), which is connected to the functional conductor (26), and also
a control unit, which is connected to the voltage or current source or the adjustable terminating impedance and is designed to control a voltage that is to be applied to the functional conductor by the voltage source or a current that is to be applied to the functional conductor by the current source or to adjust the terminating impedance, and also
an interference field sensor, which is connected to the control unit and is designed to detect a possible electromagnetic or magnetic alternating field and to deliver an output signal, corresponding to the detected electromagnetic or magnetic alternating field, to the control unit,
**characterised in that** a detector is placed at the distal end of the connected, elongate functional conductor (26) and is connected to the control unit and designed to deliver a signal regarding the heating of the distal end of the functional conductor (26) to the control unit,
wherein the control unit is designed to control the voltage or current source depending on the output signal of the interference field sensor and depending on the detector placed at the distal end in respect of a voltage or a current that is to be applied as appropriate to the functional conductor (26) or to adjust the adjustable impedance such that a voltage at the distal end of the electrode lead (20) induced by a possible electromagnetic or magnetic alternating field is compensated for by superimposing a signal generated by the current or voltage source or reflected as a result of the adjustable impedance.

2. The device according to claim 1, **characterised in that** the adjustable impedance or the voltage source is arranged on or in the vicinity of a proximal end of the electrode lead (20).

3. The device according to claim 1 or 2, **characterised in that** the adjustable terminating impedance contains a variable capacitance diode and a PIN diode.

4. The device according to claim 1 or 2, **characterised in that** the voltage source comprises a voltage-controlled oscillator.

5. The device according to any one of claims 1 to 4, **characterised in that** the interference field sensor is designed to detect a power received at the proximal end of an electrode lead (20).

6. The device according to any one of claims 1 to 4, **characterised in that** the interference field sensor is arranged at the distal end of an electrode lead (20) and comprises a non-linear component.

7. The device according to claim 6, **characterised in that** the non-linear component is a diode.

8. The device according to any one of claims 1 to 7, **characterised in that** the interference field sensor comprises a non-linear component which generates harmonic waves when current flows or voltage is applied, and the control unit is designed to adjust the adjustable terminating impedance or the voltage source such that these harmonic waves disappear.

## Revendications

1. Appareil médical implantable de manière temporaire ou permanente présentant
un stimulateur cardiaque (10) implantable, ainsi
qu'une ligne d'électrode (20) qui comprend au moins un conducteur fonctionnel (26) électrique allongé qui est relié avec le stimulateur cardiaque pour la transmission de signaux de thérapie, ou de signaux de diagnostic, ou des deux, ainsi
qu'une source de tension ou de courant pouvant être commandée ou une impédance de terminaison réglable pour le conducteur fonctionnel (26), qui est reliée avec le conducteur fonctionnel (26), ainsi
qu'une unité de commande, qui est reliée avec la source de tension ou de courant ou avec l'impédance de terminaison réglable et est conçue pour régler une tension à mettre en oeuvre, ou un courant à mettre en oeuvre, sur le conducteur fonctionnel (26) à partir de la source de tension ou de courant, ainsi
qu'un détecteur de perturbation de champ, qui est relié avec l'unité de commande et est conçu pour capter un champ alternatif électromagnétique ou magnétique éventuellement présent et pour délivrer un signal de sortie correspondant au champ alternatif électromagnétique ou magnétique détecté à l'unité de commande,
**caractérisé en ce qu'**à l'extrémité distale du conducteur fonctionnel (26) relié allongé est placé un détecteur, qui est relié avec l'unité de commande et est conçu pour délivrer un signal concernant le réchauffement de l'extrémité distale du conducteur fonctionnel (26) à l'unité de commande,
où l'unité de commande est conçue pour commander la source de tension ou de courant en fonction du signal de sortie du détecteur de perturbation de champ et du détecteur placé à l'extrémité distale en fonction de la tension à mettre en oeuvre, ou du courant à mettre en oeuvre, éventuellement sur le conducteur fonctionnel (26) ou pour régler l'impédance réglable de sorte à compenser une tension à l'extrémité distale de la ligne d'électrode (20) induite par un champ alternatif électromagnétique ou magnétique éventuellement présent par une superposition d'un signal généré par la source de courant ou de tension ou réfléchi en conséquence de l'impédance réglable.

2. Appareil selon la revendication 1, **caractérisé en ce que** l'impédance réglable ou la source de tension est disposée à une extrémité proximale de la ligne d'électrode (20) ou à proximité.

3. Appareil selon la revendication 1 ou 2, **caractérisé en ce que** l'impédance de terminaison réglable contient une diode de capacité ou une diode PIN.

4. Appareil selon la revendication 1 ou 2, **caractérisé en ce que** la source de tension présente un oscillateur commandé par la tension.

5. Appareil selon l'une des revendications 1 à 4, **caractérisé en ce que** le détecteur de perturbations de champ est conçu pour détecter une puissance reçue à l'extrémité proximale d'une ligne d'électrode (20).

6. Appareil selon l'une des revendications 1 à 4, **caractérisé en ce que** le détecteur de perturbations de champ est disposé à l'extrémité distale d'une ligne d'électrode (20) et présente un composant non linéaire.

7. Appareil selon la revendication 6, **caractérisé en ce que** le composant non linéaire est une diode.

8. Appareil selon l'une des revendications 1 à 7, **caractérisé en ce que** le détecteur de perturbation de champ présente un composant non linéaire qui génère des harmoniques lors d'un passage de courant ou d'une tension appliquée et l'unité de commande est conçue pour régler l'impédance de terminaison ou la source de tension de telle manière que ces harmoniques disparaissent.
